# EUROPÄISCHE PATENTANMELDUNG

(11) **EP 1 738 787 A1**
(43) Veröffentlichungstag der Anmeldung: **03.01.2007**
(21) Anmeldenummer: 06013207.3
(22) Anmeldetag: 27.06.2006
(51) Int. Cl.: A61M 16/04

(54) **Trachealkanüle mit Haltemittel**

(30) Priorität: 27.06.2005 DE 202005010220 U
(71) Anmelder: PRIMED HALBERSTADT MEDIZINTECHNIK GMBH, 38820 Halberstadt (DE)
(72) Erfinder: Klimenta, Klaus, 38835 Zilly (DE); Leibitzki, Harry, 38889 Blankenburg (DE); Fahl, Andreas, 51149 Köln (DE)
(74) Vertreter: Bock, Gerhard

(57) **Zusammenfassung**

Die Erfindung betrifft eine Trachealkanüle mit Haltemittel, insbesondere zum Haltern von Sicherungsketten von Sprechventilen.

Die Aufgabe der Erfindung, eine Trachealkanüle, bestehend aus Außen- und Innenrohr mit Sicherungsketten und Sprechventil anzugeben, die die Nachteile des Standes der Technik vermeidet und ein leichtes Entfernen des Sprechventils vom Patienten ermöglicht, ohne die Kette lösen bzw. die gesamte Tracheostomaprothese / Trachealkanüle entfernen zu müssen, anzugeben, wird dadurch gelöst, dass eine Trachealkanüle aus einem Innen-, einem Außenrohr und einer Sicherungskette mit Sprechventil besteht, wobei die Sicherungskette an einem Haltemittel, das Bestandteil des distalen Endes des Innenrohrs ist, befestigbar ist.

## Beschreibung

Die Erfindung betrifft eine Trachealkanüle mit Haltemittel, insbesondere zum Haltern von Sicherungsketten von Sprechventilen.

Verschiedenste Bauformen von Trachealkanülen (Tracheostomaprothesen) sind seit Jahrzehnten bekannt.

Die Schrift DE 101 09 935.5-09 offenbart bspw. eine Tracheostomaprothese, die aus einem Sprechventil mit einer Klappe, einer Außenkanüle, die mit einem sie ringförmig umgebenden Dichtballon, der über einen Luftkanal, welcher innerhalb der Außenkanüle verläuft, mit einem Druckballon verbunden ist, und die mit Perforierungen, die sich zwischen dem Dichtballon und dem Sprechventil nahe dem Dichtballon befinden, versehen ist, einer Innenkanüle mit einer Ausnehmung, die sich gegenüber den Perforierungen versetzt, nahe dem Sprechventil befindet, einer Dichtung, die zwischen der Innenkanüle und der Außenkanüle nahe dem Dichtballon dichtend angeordnet ist, und einer Absaugeinrichtung, die mit einem Absaugkanal oberhalb des Sprechventils verbunden ist, besteht.

Aus der Schrift DE 20 2004 013 323 U1 ist ein Tracheostoma-Platzhalter, bestehend aus einem rohrförmigen Tracheostoma-Schenkel, der einseitig aus einem Stück mit einem angespritzten und gewölbt ausgebildeten Tracheal-Schenkel ausgebildet ist und einem auf dem Tracheostoma-Schenkel angespritzten, angeklebten oder verschiebbaren sowie arretierbaren Flansch mit einer mehrteiligen Klemmhülse für formschlüssige oder stoffschlüssige Verbindung mit einem Aufnahmering zur Montage von Sprechventilen und künstlichen Nasen, bekannt.

Der Nachteil aller bekannten Tracheostomaprothesen / Trachealkanülen ist, dass die Sicherungsketten von Sprechventilen dazu führen, dass ein Dekonnektieren nicht ohne Lösen der Sicherungskette, die vom Außenrohr der Tracheostomaprothese / Trachealkanüle gehaltert wird, möglich ist.
Dies führt dazu, dass die gesamte Tracheostomaprothese/Trachealkanüle mit dem Sprechventil entfernt werden muss oder, dass die Sicherungskette mit einem gewissen manuellen Aufwand gelöst werden muss, um das Sprechventil vom Patienten, bspw. zum Reinigen, entfernen zu können.

Der Erfindung liegt daher die Aufgabe zugrunde, eine Trachealkanüle, bestehend aus Außen- und Innenrohr mit Sicherungsketten und Sprechventil anzugeben, die die Nachteile des Standes der Technik vermeidet und ein leichtes Entfernen des Sprechventils vom Patienten ermöglicht, ohne die Kette lösen bzw. die gesamte Tracheostomaprothese / Trachealkanüle entfernen zu müssen.

Gemäß der Erfindung wird diese Aufgabe durch die kennzeichnenden Merkmale des ersten Schutzanspruchs gelöst und durch vorteilhafte Ausgestaltungen gemäß den Unteransprüchen ergänzt.

Das Wesen der Erfindung besteht darin, dass die Trachealkanüle aus einem Innen-, einem Außenrohr und einer Sicherungskette mit Sprechventil aufgebaut ist, wobei die Sicherungskette an einem Haltemittel, das Bestandteil des distalen Endes des Innenrohrs ist, befestigbar ist.

Das Haltemittel als Bestandteil des Innenrohrs kann dabei einstückig oder zweistückig sein.

Besonders vorteilhaft ist das Haltemittel lösbarer Bestandteil des Innenrohres.

Dabei vorteilhaft ist das Haltemittel als ringförmige Kappe mit Öse zur Aufnahme des Kettenendes ausgebildet, wobei diese Kappe mit einer Verriegelung am distalen Ende des Innenrohrs befestigbar ist.

Die Öse kann sich einstückig, als Bestandteil der Kappe an dieser befinden oder zweistückig, an der Kappe gehaltert sein.

Die Kappe und / oder Öse kann aus Kunststoff und / oder Metall bestehen.

Die Öse kann an die Kappe angespritzte, angeklebten oder in diese eingeschraubt sein, so dass sie formschlüssig und / oder stoffschlüssig mit dieser verbunden ist.

Die Erfindung wird nachstehend an Hand der schematischen Zeichnung näher erläutert. Es zeigt:

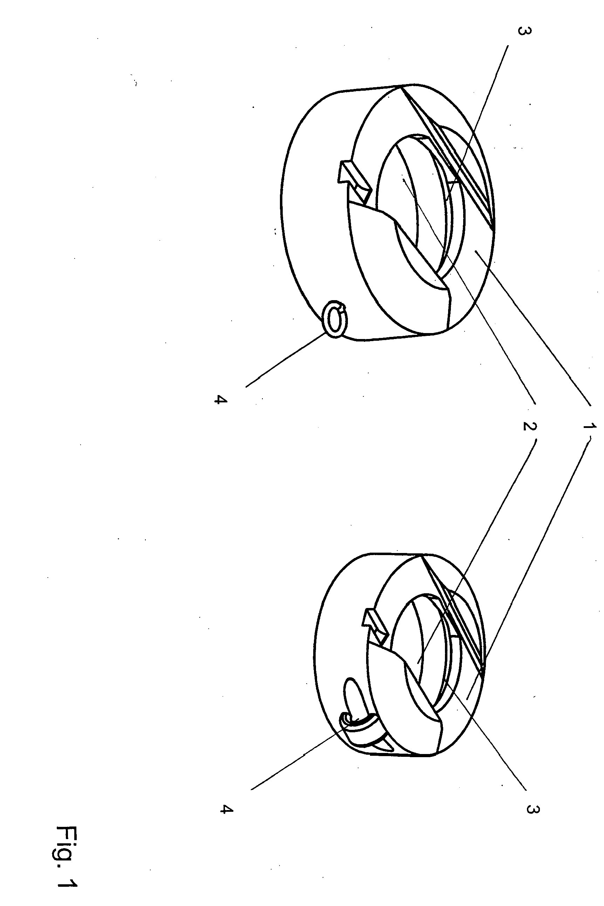

Fig. 1: eine Ausführungsform ringförmigen Kappe der erfindungsgemäßen Trachealkanüle in schematischer Darstellung.

Die in Fig. 1 dargestellte ringförmige Kappe (1) weist eine Ausnehmung (2), eine Verriegelung (3) und eine Öse (4) auf.

Vermittels der Verriegelung (3) ist die Kappe (1) mit dem Innenrohr einer Trachealkanüle verbindbar. Gleichzeitig ist die Kappe (1) an ihrer, dem Innenrohr abgewanden Seite mit einem Zubehörteil, wie bspw. einem Sprechventil verbindbar

Vermittels der Öse (4), die sich einstückig, als Bestandteil der Kappe (1) an dieser befindet oder zweistückig, an der Kappe (1) befestigt ist, ist die Sicherungskette halterbar und ist im befestigten Zustand an der Öse (4) über diese fest mit der Kappe (1) verbunden und somit fest vermittels der Kappe (1) mit dem Innenrohr verbindbar. Durch diese Sicherungskette sind diverse Zubehörteile, die mit der Kappe (1) verbindbar sind, wie bspw. Sprechventile, sicherbar, in dem die Zubehörteile beim Lösen von der Kappe (1) an der Sicherungskette hängen und vor Verlust geschützt sind.

Der Vorteil der erfindungsgemäßen Trachealkanüle ist, dass ein Dekonnektieren ohne lösen der Sicherungskette durch Trennen des Innenrohrs von Außenrohrs erfolgen kann, wobei das Außenrohr im Patienten verbleibt.

Alle in der Beschreibung, den nachfolgenden Ansprüchen und der Zeichnung dargestellten Merkmale können sowohl einzeln als auch in beliebiger Kombination miteinander erfindungswesentlich sein.

### Bezugszeichenliste

- 1 -: Kappe
- 2 -: Ausnehmung
- 3 -: Verriegelung
- 4 -: Öse

## Patentansprüche

1. Trachealkanüle bestehend aus einem Innen-, einem Außenrohr und einer Sicherungskette mit Sprechventil, wobei die Sicherungskette an einem Haltemittel, das Bestandteil des distalen Endes des Innenrohrs ist, befestigbar ist.

2. Trachealkanüle gemäß Schutzanspruch 1, **dadurch gekennzeichnet, dass** das Haltemittel Bestandteil des einstückigen Innenrohrs ist.

3. Trachealkanüle gemäß Schutzanspruch 1, **dadurch gekennzeichnet, dass** das Haltemittel am distalen Ende des Innenrohrs halterbar ist.

4. Trachealkanüle gemäß Schutzanspruch 2 oder 3, **dadurch gekennzeichnet, dass** das Haltemittel fester Bestandteil des Innenrohres ist.

5. Trachealkanüle gemäß Schutzanspruch 2 oder 3, **dadurch gekennzeichnet, dass** das Haltemittel lösbarer Bestandteil des Innenrohres ist.

6. Trachealkanüle gemäß einem der voran stehenden Schutzansprüche, **dadurch gekennzeichnet, dass** das Haltemittel als ringförmige Kappe mit Öse zur Aufnahme des Kettenendes ausgebildet ist.

7. Trachealkanüle gemäß Schutzanspruch 6, **dadurch gekennzeichnet, dass**, die Kappe mit einer Verriegelung am distalen Ende des Innenrohrs befestigbar ist.

8. Trachealkanüle gemäß Schutzanspruch 6, **dadurch gekennzeichnet, dass** sich die Öse einstückig, als Bestandteil der Kappe an dieser befindet.

9. Trachealkanüle gemäß Schutzanspruch 6, **dadurch gekennzeichnet, dass** die Öse zweistückig, an der Kappe gehaltert ist.

10. Trachealkanüle gemäß Schutzanspruch 8 oder 9, **dadurch gekennzeichnet, dass** Öse an die Kappe angespritzt, angeklebte oder in diese eingeschraubt ist, so dass sie formschlüssig und / oder stoffschlüssig mit dieser verbunden ist.

11. Trachealkanüle gemäß einem der voran stehenden Schutzansprüche, **dadurch gekennzeichnet, dass** die Kappe und / oder Öse aus Kunststoff und / oder Metall bestehen.
